Europäisches Patentamt

European Patent Office

Office européen des brevets

(19)

(11) Publication number: **0 271 204**

**A2**

(12) **EUROPEAN PATENT APPLICATION**

(21) Application number: 87309724.0

(22) Date of filing: 03.11.87

(51) Int. Cl.4: **G01N 33/558** , G01N 33/544 ,
G01N 33/94

(30) Priority: 07.11.86 US 928771

(43) Date of publication of application:
15.06.88 Bulletin 88/24

(84) Designated Contracting States:
AT BE CH DE ES FR GB IT LI LU NL SE

(71) Applicant: SYNTEX (U.S.A.) INC.
3401 Hillview Avenue P.O. Box 10850
Palo Alto California 94303(US)

(72) Inventor: Ullman, Edwin F.
135 Selby Lane
Atherton California 94025(US)
Inventor: Ghazarossian, vartan
340 Olive Street
Menlo Park California 94025(US)
Inventor: Peries, Rohan
108 Dalma Drive
Mountain View California 94041(US)
Inventor: Sizto , Ning Chung
332 Ely Place
Palo Alto California 94306(US)

(74) Representative: Armitage, Ian Michael et al
MEWBURN ELLIS & CO. 2/3 Cursitor Street
London EC4A 1BQ(GB)

(54) Immunochromatographic method and device.

(57) A method for determining the presence of an analyte in a sample suspected of containing the analyte is disclosed. The analyte is a member of a specific binding pair ("sbp member") consisting of ligand and its complementary receptor. The method comprises contacting, with a test solution containing said sample and a first sbp member analogous to said analyte, a contact portion of a piece of bibulous material capable of being traversed in at least one direction by said test solution by capillary migration. The bibulous material contains a second sbp member capable of binding the analyte and the first sbp member. The second sbp member is non-diffusively bound to the bibulous material at least at a portion thereof between the contact portion and a small situs on the piece separated from the contact portion. The surface area of the situs is substantially less than that of the piece of bibulous material. the situs is capable of binding the first sbp member not bound to said second sbp member. Next, at least a portion of the test solution is allowed to traverse the bibulous material by means of capillary migration and thereby contact the situs. The situs is examined for the presence of the first sbp member at the situs, which is usually indicated by the presence of a detectible signal. Such signal can be directly detectible, or the situs can be exposed to a signal producing means capable of interacting with the first sbp member to produce a detectible signal at the situs in relation to the amount of analyte in the sample. The signal at the situs is distinguishable from signal detectible at portions of the bibulous material other than the situs.

-1-

# IMMUNOCHROMATOGRAPHIC METHOD AND DEVICE

The ability to employ naturally occurring receptors or antibodies directed to specific compounds in assaying for the presence of a compound of interest has created a burgeoning immunoassay business. In each of the assays, a homologous pair of specific binding pair ("sbp") members, usually an immunological pair, involving a ligand and a receptor (antiligand) is involved, wherein one of the sbp members is labeled with a label which provides a detectible signal. The immunoassay methodology results in a distribution of the signal label between signal label bound in a complex of the sbp members and unbound signal label. The differentiation between bound and unbound signal label can be as a result of physical separation of bound from unbound signal label or modulation of the detectible signal between bound and unbound signal label.

For the most part, immunoassays have been directed to quantitative determination of a wide variety of compounds of interest in clinical laboratories requiring relatively sophisticated equipment and careful

5687H                                          25780-FF

- 2 -

technique. Immunoassays have found less extensive commercial application where semi-quantitative or qualitative results would be acceptable and the determination would involve non-laboratory personnel, such as in a home or a medical practitioner's office. Even in the clinical laboratory, simple and rapid screening tests employing inexperienced personnel could serve to provide substantial economies.

In developing an immunoassay, there are many considerations. One consideration is to provide substantial differentiation between the observed signal resulting from signal label when bound as compared to unbound. Another consideration is to minimize interference from endogenous materials in the sample suspected of containing the compound of interest. A further consideration is the ease with which the observed signal can be detected and serve to differentiate between concentrations in the concentration range of interest. Other factors include the ease of preparation of the reagents, the precision with which samples and reagent solutions must be prepared and measured, the storage stability of the reagents, the number of steps required in the protocol, and the proficiency and accuracy with which each of the steps must be performed. Therefore, in developing an assay that can have application with untrained personnel, such as assays to be performed in the home, in forensic medicine, by medical practitioners, or the like, the observed result should be minimally affected by variations in the manner in which the protocol is carried out and the techniques for performing the various steps should be simple.

In general, immunoassays that permit the simultaneous determination of two or more analytes have been difficult to design and those that have been

5687H

demonstrated utilize different radioactive labels on separate analyte analogs.

A test device for determining a characteristic of a sample, particularly for determining substances in fluid samples, is disclosed in U.S. Patent No. 4,094,647. A thin layer chromatography device and method of making a chromatography test is disclosed in U.S. Patent No. 4,384,958. An immunoassay wherein labeled antibody is displaced from immobilized analyte analog is described in U.S. Patent No. 4,434,236. A device and method for detecting myoglobin is disclosed in U.S. Patent No. 4,189,304. Test strips for analyzing substances dissolved in liquids are described in U.S. Patent No. 4,438,067. A multi-layered test device for determining the presence of a liquid sample component and the method of using such a device, are described in U.S. Patent No. 4,160,008. A method for measuring antigen by labeled antigen using insoluble antibody is disclosed in Japanese Patent Application Laid-Open No. 5925/73 - January 25, 1973.

A concentrating zone method in heterogeneous immunoassays is disclosed in U.S. Patent No. 4,366,241. U.S. Patent No. 4,168,146 describes an immunoassay test strip. U.S. Patent Nos. 3,990,850 and 4,055,394 describe diagnostic test cards. An automated method for quantitative analysis of biological fluids is described in U.S. Patent No. 4,327,073. A chromogenic support immunoassay is disclosed in International Application No. PCT/US83/01887.

A wide variety of patents and patent applications provide an extensive literature of different techniques for producing detectible signals in immunoassays. The following list is merely illustrative of some of these techniques which can find application in this invention.

The following is a list of United States patents and patent applications and a general statement of the type of label involved:

U.S. Patent Nos. 3,646,346, Radioactive Label; 3,654,090, 3,791,932 and 3,817,838, Enzyme Labels; 3,996,345, Fluorescer-Quencher Labels; 4,062,733, Radioactive Label; 4,067,959, Fluorescer or Enzyme Label; 4,104,029, Chemiluminescent Label; and 4,160,645, Non-Enzymatic Catalyst Label. See U.S. Patent Nos. 3,966,879 for an electrophoretic technique employing an antibody zone and 4,120,945 for an RIA where labeled analyte is initially bound to a solid support through antibody. U.S. Patent No. 4,233,402 employs enzyme pair labels; U.S. Patent No. 4,720,450, chemically induced fluorescent labels; and U.S. Patent No. 4,287,300, enzyme anionic charge labels.

The methods and devices of the present invention are useful for determining the presence of an analyte in a sample suspected of containing the analyte particularly when a plurality of analytes are present which must be individually identified. The device is a piece of bibulous material capable of being traversed in at least one direction by a test solution through capillary migration. The test solution is comprised of the sample and a first sbp member analogous to the analyte. The bibulous material contains a second sbp member non-diffusively bound thereto at least at a portion thereof between a contact portion and a small situs on the piece separated from the contact portion. The second sbp member is capable of binding the analyte and the first sbp member. The situs is capable of binding the first sbp member not bound to the second sbp member. Usually, a binding agent such as the second sbp member or other receptor for the first sbp member is non-diffusively bound to the bibulous material at the situs at a density higher than that at portions of the piece adjacent to the situs.

In the method the contact portion of the piece of bibulous material separated from the situs is contacted with the above test solution, which traverses the bibulous material by means of capillary action. At least a portion of the test solution is allowed to traverse the bibulous material and contact the situs. The situs is examined for the presence of the first sbp member, which is usually indicated by the presence of a detectible signal at the situs. Such signal can be detected directly or the situs can be exposed to a signal producing means capable of interacting with the first sbp member to produce a detectible signal in relation to the amount of analyte in the test solution. The signal is detected at the situs and is distinguishable from signal detectible at portions of the bibulous material other than the situs. The presence of a signal at one or more of the situses indicates the presence and identity of one or more of the analytes in the sample.

In one embodiment of the present invention the signal produced at the small situs has a sharp-edged distinctive pattern that provides a sharp contrast to the signal produced at portions of the piece other than at the situs when analyte is present in the test solution.

In another embodiment of the present invention the binding agent is non-diffusively bound to the small situs on the piece of bibulous material through the intermediacy of particles non-diffusively bound to the small situs.

The method and device of the present invention are advantageous because the device and methods are simple to use and can be applied to a plurality of analytes in a single test solution or multiple test solutions. The presence or absence, and identity, of one or more analytes in the test solution can be readily determined using a single piece of bibulous material and appropriate first and second sbp members. Usually a separate situs will be provided for each analyte each capable of binding a separate first sbp member. In addition, the method of the invention provides for the detection of analytes, such as drugs, without the need for reference materials or instrumentation.

As mentioned above, the present invention is directed to methods and devices for determining the presence at or above a predetermined minimum detectible amount of one or more analytes in a sample suspected of containing one or more of the analytes. A test solution is formed by combining in an aqueous medium the sample and a first sbp member analogous to the analyte, usually a conjugate of the analyte and a label. A portion, i.e., the "contact portion", usually an end portion of a piece, usually a strip, of bibulous material capable of being traversed in at least one direction by this test solution by means of capillary migration is contacted with the test solution. The bibulous material contains a second sbp member capable of binding to the analyte and the first sbp member. The second sbp member is non-diffusively, and preferably uniformly, bound to the bibulous material at least at a portion thereof between the contact portion and a small situs separated from the contact portion. The surface area of the situs is substantially less than that of the piece of bibulous material. The situs is capable of binding the first sbp member not bound to the second sbp member. At least a portion of the test solution is allowed to traverse the bibulous material by capillary action and thereby contact the situs. Next, the first sbp member bound to the situs is detected. Detection may be achieved directly, for example, when the first sbp member is labeled with a radio-active label, or the situs is exposed to a signal producing means such as light, heat or a chemical reagent capable of interacting with the label to produce a signal in relation to the amount of analyte in the test solution. The signal produced at the situs is then detected. In the presence of analyte in the sample, the signal is distinguishable from signal detectible at a portion of the bibulous material adjacent to the situs. The signal producing means is reactive with the first sbp member and includes reagents required to produce a detectible signal at the situs in relation to the presence at or above a predetermined amount of analyte in the sample.

A binding agent, usually a specific binding partner that can bind the first sbp member, is non-diffusively bound to the situs. In one embodiment of the present invention the binding agent is the second sbp member which is conjugated to particles. The particles are non-diffusively bound to the bibulous material at the situs to provide a high density of the binding agent at the situs. The situs can be a narrow or wide band running transverse to the direction of traversal of the test solution along the piece of bibulous material. The signal produced at the situs can be a narrow or wide band, a sharp-edged distinctive pattern, or the like. The signal generated at the situs will usually be measured in comparison with the signal produced at adjacent areas on the piece of bibulous material. Frequently, visual detection will be used. On the other hand, for very sensitive assays or when there are a multiplicity of situses the level of signal can be measured instrumentally.

The present invention can be applied to the determination of the presence and identity of one or more of a plurality of analytes in a test solution.

Before proceeding further with the description of the specific embodiments of the present invention, a number of terms will be defined.

Analyte--the compound or composition to be measured that is capable of binding specifically to an antibody, usually an antigen or drug.

The precise nature of the antigenic and drug analytes together with numerous examples thereof are disclosed in U.S. Patent 4,299,916 to Litman, et al., particularly columns 16 to 23, and in U.S. Patent No. 4,275,149, columns 17 and 18, the disclosures of which are incorporated herein by reference.

The analytes are characterized by having single binding sites (monovalent) or multiple binding sites (polyvalent). The polyvalent analytes will normally be poly(amino acids), i.e., polypeptides and proteins, polysaccharides, nucleic acids, and combinations thereof. Such combinations or assemblages include bacteria, viruses, chromosomes, genes, mitochondria, nuclei, cell membranes, and the like.

A wide variety of proteins may be considered as to the family of proteins having similar structural features, proteins having particular biological functions, proteins related to specific microorganisms, particularly disease causing microorganisms, etc.

The monoepitopic ligand analytes will generally be from about 100 to 2,000 molecular weight, more usually from 125 to 1,000 molecular weight. The analytes of interest include drugs, metabolites, pesticides,. pollutants, and the like. Included among drugs of interest are the alkaloids. Among the alkaloids are morphine alkaloids, which includes morphine, codeine, heroin, dextromethorphan, their derivatives and metabolites; cocaine alkaloids, which include cocaine and benzoyl ecgonine, their derivatives and metabolites, ergot alkaloids, which include the diethylamide of lysergic acid; steroid alkaloids; iminazoyl alkaloids; quinazoline alkaloids, isoquinoline alkaloids; quinoline alkaloids, which include quinine and quinidine; diterpene alkaloids, their derivatives and metabolites.

The next group of drugs includes steroids, which includes the estrogens, estrogens, androgens, andreocortical steroids, bile acids, cardiotonic glycosides and aglycones, which includes digoxin and digoxigenin, saponins and sapogenins, their derivatives and metabolites. Also included are the steroid mimetic substances, such as diethylstilbestrol.

The next group of drugs is lactams having from 5 to 6 annular or ring members, which include the barbituates, e.g. phenobarbital and secobarbital, diphenylhydantonin, primidone, ethosuximide, and their metabolites.

The next group of drugs is aminoalkylbenzenes, with alkyl of from 2 to 3 carbon atoms, which includes the amphetamines, catecholamines, which includes ephedrine, L-dopa, epinephrine, narceine, papaverine, and their metabolites.

The next group of drugs is benzheterocyclics which include oxazepam, chlorpromazine, tegretol, imipramine, their derivatives and metabolites, the heterocyclic rings being azepines, diazepines and phenothiazines.

The next group of drugs is purines, which includes theophylline, caffeine, their metabolites and derivatives.

The next group of drugs includes those derived from marijuana, which includes cannabinol and tetrahydrocannabinol.

5687H

The next group of drugs includes the vitamins such as A, B, e.g., $B_{12}$, C, D, E and K, folic acid, and thiamine.

The next group of drugs is prostaglandins, which differ by the degree and sites of hydroxylation and unsaturation.

The next group of drugs is antibiotics, which include penicillin, chloromycetin, actinomycetin, tetracycline, terramycin, the metabolites and derivatives.

The next group of drugs is the nucleosides and nucleotides, which include ATP, NAD, FMN, adenosine, guanosine, thymidine, and cytidine with their appropriate sugar and phosphate substituents.

The next group of drugs is miscellaneous individual drugs which include methadone, meprobamate, serotonin, meperidine, amitriptyline, nortriptyline, lidocaine, procaineamide, acetylprocaineamide, propranolol, griseofulvin, valproic acid, butyrophenones, antihistamines, anticholinergic drugs, such as atropine, their metabolites and derivatives.

Metabolites related to diseased states include spermine, galactose, phenylpyruvic acid, and porphyrin Type 1.

The next group of drugs is aminoglycosides, such as gentamicin, kanamicin, tobramycin, and amikacin.

Among pesticides of interest are polyhalogenated biphenyls, phosphate esters, thiophosphates, carbamates, polyhalogenated sulfenamides, their metabolites and derivatives.

For receptor analytes, the molecular weights will generally range from 10,000 to $2 \times 10^8$, more usually from 10,000 to $10^6$. For immunoglobulins, IgA, IgG, IgE and IgM, the molecular weights will generally vary from about 160,000 to about $10^6$. Enzymes will normally range from about 10,000 to 1,000,000 in molecular weight. Natural receptors vary widely, generally being at least about 25,000 molecular weight and may be $10^6$ or higher molecular weight, including such materials as avidin, DNA, RNA, thyroxine binding globulin, thyroxine binding prealbumin, transcortin, etc.

Member of a specific binding pair ("sbp member") -- one of two different molecules having an area on the surface or in a cavity which specifically binds to and is thereby defined as complementary with a particular spatial and polar organization of the other molecule. The members of the specific binding pair are referred to as ligand and receptor (antiligand). These will usually be members of an immunological pair such as antigen-antibody, although other specific binding pairs such as biotin-avidin hormones-hormone receptors, nucleic acid duplexes, IgG-protein A, DNA-DNA, DNA-RNA, and the like are not immunological pairs but are included in the definition.

Ligand -- any organic compound for which a receptor naturally exists or can be prepared.

Receptor ("antiligand") -- any compound or composition capable of recognizing a particular spatial and polar organization of a molecule, e.g., epitopic or determinant site. Illustrative receptors include naturally occurring receptors, e.g., thyroxine binding globulin, antibodies, enzymes, Fab fragments, lectins, nucleic acids, protein A, complement component Clq. and the like.

Labeled sbp member -- a label, generally capable of electrochemical detection or absorption or emission of electromagnetic radiation, a catalyst, frequently an enzyme, bound to a first sbp member. The labeled sbp member is a member of the signal producing system and the first sbp member is chosen to bind to the second sbp member in accordance with a particular protocol in an assay.

Antibody -- an immunoglobin, or derivative or fragment thereof, having an area on the surface or in a cavity which specifically binds to and is thereby defined as complementary with a particular spatial and polar organization of another molecule. The antibody can be monoclonal or polyclonal and can be prepared by techniques that are well known in the art such as, for example, immunization of a host and collection of sera or hybrid cell line technology.

Antibody for the analyte -- an antibody specific for an analyte.

First sbp member -- a modified analyte or analyte analog or surrogate which can compete with the analogous analyte in binding to a second sbp member, usually a receptor or antibody, the modification providing means to join the analyte analog to a label to provide a labeled sbp member. The analyte analog will usually differ from the analyte by more than replacement of a hydrogen with a bond which links the analyte analog to a hub or label, but need not. The term analyte surrogate refers to a compound having the capability of binding the antibody for the analyte. Thus, the analyte surrogate may bind to the antibody for the analyte in a manner similar to the analyte. On the other hand, the surrogate could be, for example, an antibody directed against the idiotype of an antibody to the analyte.

The first sbp member can also be a conjugate of an analyte, a label, e.g., enzyme, and a third sbp member. The third sbp member will be a compound other than one of the analytes suspected of being present in the sample. The third sbp member can be, for example, a ligand such as a hapten or low molecular weight compound, e.g., a drug, fluorescein, biotin, and the like. The advantage of this particular embodiment is that the same enzyme can be used in each conjugate. Thus, the use of a plurality of enzymes and corresponding signal producing systems is avoided.

Second sbp member -- an sbp member capable of binding to the analyte and the first sbp member. The second sbp member can bind to a determinant site on the analyte and to a determinant site on the first sbp member. A preferred second sbp member is an antibody.

Binding agent -- a material non-diffusively bound to the situs capable of binding the first sbp member. The material may bind non-specifically or specifically. When the binding is non-specific, the binding agent will usually be hydrophobic or polyionic. When the binding agent provides for specific binding, it will usually be an sbp member complementary to the labeled sbp member, preferably a receptor for the first sbp member. As a practical matter, the binding agent will usually be the second sbp member, but it may be either member of a large variety of specific binding pairs, provided only that the complementary member is bound to the labeled sbp member. When the first sbp member is a conjugate comprising an analyte, a label, and a third sbp member, the binding agent can be a corresponding receptor for the third sbp member.

Bibulous material -- a porous material having pores of at least 0.1μ, preferably at least 1.0μ, which is susceptible to traversal by an aqueous medium in response to capillary force. Such materials are generally hydrophilic or are capable of being rendered hydrophilic and include inorganic powders such as silica, magnesium sulfate, and alumina; natural polymeric materials, particularly cellulosic materials and materials derived from cellulose, such as fiber containing papers, e.g., filter paper, chromatographic paper, etc.; synthetic or modified naturally occurring polymers, such as nitrocellulose, cellulose acetate, poly (vinyl chloride), polyacrylamide, cross linked dextran, agarose, polyacrylate, etc.; either used by themselves or in conjunction with other materials; ceramic materials; and the like. The bibulous material can be attached to a support. On the other hand, the bibulous material may provide its own support. The bibulous material may be polyfunctional or be capable of being polyfunctionalized to permit covalent bonding of receptors or antibodies as well as to permit bonding of other compounds which form a part of the signal producing system.

Binding of receptors and antibodies to the bibulous material may be accomplished by well-known techniques, commonly available in the literature. See, for example, "Immobilized Enzymes," Ichiro Chibata, Halsted Press, New York (1978) and Cuatrecasas, J. Bio. Chem., 245:3059 (1970).

The piece of bibulous material can be a single structure such as a sheet cut into strips or it can be several strips or particulate material bound to a support or solid surface such as found, for example, in thin-layer chromatography and may have an absorbent pad either as an integral part or in liquid contact. The piece of bibulous material can be comprised of several segments, one or more being an absorbent pad, bound to a support. The piece of bibulous material can also be a sheet having lanes thereon or capable of spotting to induce lane formulation, wherein a separate assay can be conducted in each lane. The absorbent pad may be any hydrophilic bibulous material such as paper, sponge, felt, porous polymers and the like. The piece of bibulous material can have a rectangular, circular, oval, triagonal or other shape provided that there is at least one direction of traversal of a test solution by capillary migration. Other directions of traversal may occur such as in an oval or circular piece contacted in the center with the test solution. However, the main consideration is that there be at least one direction of flow to a situs. In the following discussion strips of bibulous material will be described by way of illustration and not limitation.

The support for the bibulous material, where a support is desired or necessary, will normally be water insoluble, non-porous, and rigid and usually will be of the same length and width as the bibulous strip but may be larger or smaller. A wide variety of organic and inorganic materials, both natural and synthetic, and combinations thereof, may be employed provided only that the support does not interfere with the capillary action of the strip, or non-specifically bind assay components, or interfere with the signal producing system. Illustrative polymers include polyethylene, polypropylene, poly(4-methylbutene), polystyrene, polymethacrylate, poly(ethylene terephthalate), nylon, poly(vinyl butyrate), glass, ceramics, metals, and the like.

Label -- A label may be any molecule bound to the first member that is required to produce a signal. In the subject invention, the label may be inert and serve solely as a binding site for a member of the signal producing means or it may spontaneously produce a detectable signal or may produce a detectable signal in conjunction with a signal producing means. The label may be isotopic or nonisotopic, preferably nonisotopic. However, an isotopic label can be preferred for achieving high sensitivity when using radio-autographic detections with photographic film.

Signal producing means -- means capable of interacting with the label to produce a detectible signal. Such means include, for example, electromagnetic radiation, heat, chemical reagents, and the like. Where chemical reagents are employed, some of the chemical reagents can be included as part of a developer solution. The chemical reagents can include substrates, coenzymes, enhancers, second enzymes, activators, cofactors, inhibitors, scavengers, metal ions, specific binding substances required for binding of signal generating substances, and the like. Some of the chemical reagents such as coenzymes, substances that react with enzymic products, other enzymes and catalysts, and the like can be bound to the strip.

Signal producing system -- The signal producing system may have one or more components, at least one component being the labeled sbp member. The signal producing system includes all of the reagents required to produce a measurable signal including signal producing means capable of interacting with the label to produce a signal.

The signal producing system provides a signal detectable by external means, normally by measurement of electromagnetic radiation, desirably by visual examination. For the most part, the signal producing system includes a chromophoric substrate and enzyme, where chromophoric substrates are enzymatically converted to dyes which absorb light in the ultraviolet or visible region, phosphors or fluorescers.

The signal-producing system can include at least one catalyst as a label, usually at least one enzyme, and at least one substrate and may include two or more catalysts and a plurality of substrates, and may include a combination of enzymes, where the substrate of one enzyme is the product of the other enzyme. The operation of the signal producing system is to produce a product which provides a detectable signal at the situs, related to the presence of label bound to the situs, as a result of the binding of the conjugate to the situs by means of the first receptor.

Two catalysts may be employed, either a combination of an enzyme and a non-enzyme catalyst or two enzymes, where the two catalysts are related in that the product of one is the substrate of the other. In this system, there need be only one substrate which can undergo successive changes catalyzed by the catalysts, which results in the compound involved with production of a detectable signal. For the most part, however, there will normally be a substrate for the first enzyme in the series and a second compound, which serves as a precursor to the compound involved in the production of the signal, normally providing the compound which produces the signal. Thus, the product of the first enzyme may react with the precursor to the compound that produces a signal to provide the compounds that generates the signal.

Where two enzymes are employed, the involved reactions will be, for the most part, hydrolysis or redox reactions. In the case of hydrolysis, a derivatized dye precursor that has a hydrolytically labile bond, the hydrolytic enzyme and an enzyme that catalyzes the conversion of the released dye precursors to a dye conversion product is illustrative of this type of system. In redox reactions, a first enzyme can produce an essential oxidizing substrate required for the second enzyme, where the second enzyme catalyzes the reaction between the oxidizing substrate and a dye precursor.

Where two enzymes are used, the first enzymatic reaction may involve hydrolytic cleavage or a redox reaction of the substrate to provide a product which is the substrate of another enzyme. The first situation may be illustrated by glucose-6-phosphate being catalytically hydrolyzed by alkaline phosphatase to glucose, where glucose is a substrate for glucose oxidase. The second situation may be illustrated by glucose being oxidized by glucose oxidase to provide hydrogen peroxide which would enzymatically react with a leuco dye to produce a signal generator.

Coupled catalysts can also involve an enzyme with a non-enzymatic catalyst. The enzyme can produce a reactant which undergoes a reaction catalyzed by the non-enzymatic catalyst or the non-enzymatic catalyst may produce a substrate (includes coenzymes) for the enzyme. A wide variety of non-enzymatic catalysts which may be employed are found in U.S. Patent No. 4,160,645, issued July 10, 1979, the appropriate portions of which are incorporated herein by reference.

Various combinations of enzymes may be employed to provide a signal generating compound. Particularly, combinations of hydrolases may be employed to produce an insoluble signal generator. Alternatively, combinations of hydrolases and oxidoreductases can provide the signal generating compound. Also, combinations of oxidoreductases may be used to produce an insoluble signal generating compound.

For combinations of enzymes one enzyme can be non-diffusively bound to the bibulous material, while the other enzyme is the label conjugated to the analyte. Additionally, one or more other members of the signal producing system can be bound to the bibulous material depending on the particular signal producing system chosen or the particular protocol followed.

In order to have a detectable signal, it is desirable to provide means for amplifying the signal produced by the presence of the label bound at the situs. Therefore, it will usually be preferable for the label to be a catalyst or luminescent compound or radioisotope, most preferably a catalyst. Preferably, catalysts are enzymes and coenzymes which can produce a muliplicity of signal generating molecules from a single label.

An enzyme or coenzyme is employed which provides the desired amplification by producing a product, which absorbs light, e.g., a dye, or emits light upon irradiation, e.g., a fluorescer. Alternatively, the catalytic reaction can lead to direct light emission, e.g., chemiluminescence. A large number of enzymes and coenzymes for providing such products are indicated in U.S. Patent No. 4,275,149 bridging columns 19 to 23, and U.S. Patent No. 4,318,980, columns 10 to 14, which disclosures are incorporated herein by reference.

A number of enzyme combinations are set forth in U.S. Patent no. 4,275,149, bridging columns 23 to 28, which combinations can find use in the subject invention. This disclosure is incorporated herein by reference.

Of particular interest are enzymes which involve the production of hydrogen peroxide and the use of the hydrogen peroxide to oxidize a dye precursor to a dye. Particular combinations include saccharide oxidases, e.g., glucose and galactose oxidase, or heterocyclic oxidases, such as uricase and xanthine oxidase, coupled with an enzyme which employs the hydrogen peroxide to oxidize a dye precursor, that is, a peroxidase such as horseradish peroxidase, lactoperoxidase, or microperoxidase. Additional enzyme combinations may be found in the subject matter incorporated by reference. When a single enzyme is used as a label, other enzymes may find use such as hydrolases, transferases, and oxidoreductases, preferably, hydrolases such as alkaline phosphatase and $\beta$-galactosidase. Alternatively luciferases may be used such as firefly luciferase and bacterial luciferase.

Illustrative coenzymes which find use include NAD[H]; NADP[H], pyridoxal phosphate; FAD[H]; FMN[H], etc., usually coenzymes involving cycling reactions, see particularly U.S. Patent No. 4,318,980.

The product of the enzyme reaction will usually be a dye or fluorescer. A large number of illustrative fluorescers are indicated in U.S. Patent No. 4,275,149, columns 30 and 31, which disclosure is incorporated herein by reference.

Ancillary Materials -- Various ancillary materials will frequently be employed in the assay in accordance with the present invention. For example, buffers will normally be present in the assay medium, as well as stabilizers. Frequently, in addition to these additives, additional proteins may be included, such as albumins, or surfactants, particularly, non-ionic surfactants, binding enhancers, e.g. polyalkylene glycols, or the like.

Situs -- an area on the piece of bibulous material which has a surface area less than the surface area of the piece of bibulous material. The situs may be a narrow or wide line, curve, band, or combinations thereof, or the like. Generally, the direction of traversal by the test solution on the piece of bibulous material will be transverse to the situs. In one embodiment the situs is a wide band removed from the contact portion of the bibulous material. In another embodiment the signal produced at the situs has a sharp-edged distinctive pattern that provides a sharp contrast to signal produced at portions of the piece other than the situs. For example, the situs can be a printed display of an abbreviated name or names of the analyte or analytes in the test solution, of a plus sign, or the like. The situs is separated from the portion of the piece contacted with the test solution in accordance with the principle of the present invention. The portion of the piece between the situs and the contact portion should be large enough to assure that a high percentage of the labeled sbp member becomes bound to the second sbp member and not to the situs when a test solution containing no analyte and the labeled sbp member are caused to traverse the bibulous material. At least 90%, preferably at least 99% of the labeled sbp member should become bound between reaching the situs under these conditions.

The situs is capable of binding the first sbp member not bound to the second sbp member on the piece of bibulous material between the situs and the contact portion. The signal detectible at the situs must be distinguishable from signal detectible at a portion of the piece other than the situs. Usually, this may be realized by providing a high density of binding agent, usually a receptor for the labeled sbp member, at the situs in relation to the density of the second sbp member at other portions of the piece of bibulous material. The primary requirement is that the density of the signal at the situs should be sufficiently higher than at portions of the piece immediately adjacent to the situs so that the signal at the situs is distinguishable from the adjacent signals so as to indicate the presence of analyte in the sample. Where the binding agent or second sbp member or other receptor for the first sbp member is bound to the situs, the surface density thereof should be greater than, usually at least twice, preferably at least 10 to 100 times, the amount of the second sbp member at portions of the bibulous material adjacent to the situs. Preferably, the situs will be located on a portion of the piece that has no second sbp member bound to it.

The method for creating the situs on the piece of bibulous material can vary widely. Binding agent can be formed or can be caused to bind exclusively at a portion of the piece subjected to physical or photochemical activation as, for example, by heat or light. Alternatively, a solution or suspension of a specific binding agent may be applied to a small portion of a piece that has been pretreated to cause the binding agent to adhere to the bibulous material upon contact, for example, by the use of ionic interactions or by ligand-receptor binding. Alternatively, the piece can be constructed in segments, one of which has bound to it the second sbp member and one or more others have incorporated binding agents to provide situses. More than one of the latter segments may be used provided that in the final device each has liquid contact with the segment bearing the second sbp member.

In a preferred embodiment the binding agent can be bound to particles and the particles can be non-diffusively bound to the piece at the situs.

The nature of the particles or the beads may vary widely, being naturally occurring or synthetic, The materials are commercially available or commercially available materials may be modified. Exemplary of such particles or beads are latex particles made from polystyrene, polyacrylates, polyacrylamide, available as Biogel-p, or naturally occurring materials such as polysaccharides, particularly cross-linked polysaccharides, such as agarose, which is available as Sepharose, dextran, available as Sephadex, microcrystalline cellulose, starch and the like. Other materials include polyacrylamides, polystyrene, polyvinyl alcohol, copolymers of hydroyethyl methacrylate and methyl methacrylate, silicones, glasses, available as Bioglas, diatomaceous earth, silica, and the like. The primary requirement is that the materials do not contribute a signal, usually fluorescence light absorption or scattering, that would produce a signal at the situs that is unrelated to the amount of analyte in the sample.

The particles must be capable of non-diffusable attachment to the binding agent where the attachment can be achieved by covalent or non-covalent binding. A wide varity of functional groups can be used for covalent linking including carboxylic acids, aldehydes, amines, amides, activated ethylenes such as maleimide, hydroxyls, sulfonic acids, mercaptans, and the like. The manner of linking a wide variety of compounds to the various particles is well-known and is amply illustrated in the literature, See, for example, Cautrecases, J. Biol, Chem., 245, 3059(1970). Alternatively, for non-covalent linking, particles having appropriate ligands or receptors or particles having non-specific binding properties are incubated with the binding agent.

Upon application of the particles to the bibulous material the particles should not migrate to any significant degree away from the point of application. Particle migration can be controlled in part by controlling the size of the particles. In general, the particles should be of a size that will permit them to infiltrate the pores of the bibulous material and become imbedded or non-diffusively bound therein. Thus, the particles are generally slightly larger than the minimum size of the pores of the bibulous material and smaller than the maximum pore size. Usually, the size of the particles will range from about 0.1 to 25 microns, more usually from about 0.4 to 10 microns, preferably greater than 0.5 µ.

Application of particles having a non-diffusively bound binding agent to a piece of bibulous material can provide a situs with sharply defined edges suitable for binding the first sbp member. Several methods may be employed. Usually a suspension of the particles in a liquid, which frequently is aqueous, can be applied to the bibulous material. Application may be made by any standard printing process including the use of electrostatic and laser propelled jets, and printing probe or type face. In addition, particles can be applied by template. In this situation, the shape of the situs would be defined by a cut pattern through which particles would be absorbed into the bibulous material. Alternatively, the suspension can be transferred to the bibulous material by inscribing with a pen or microcapillary tube, or by means of a fine liquid stream. Where dry particles are used, they may be applied by directing a jet of a suspension of the particles in a gas, usually air, at the desired situs. In each case, particularly when printing techniques are not used, it will frequently be desirable to provide for reduced pressure on the side of the piece opposite to the side used to apply the particles. Pressure reduction is conveniently provided by placing a sheet of the bibulous material on a filter or porous plate that covers a vacuum chamber. The suspension is then applied while air is being drawn through the material. Regardless of the method of application of the particles it is usually preferable to wash the situs free of unbound particles after they have been applied.

The liquid used to suspend the particles will usually be aqueous and must not dissolve the particles or damage or release the binding agent when the binding agent is bound to the particles prior to fixing the particles onto the piece of bibulous material. Thickners and surfactants may be added to limit capillary flow and provide sharply defined edges. Thickners may include polyvinyl alcohol, polypyrrolidone, dextran, glycerol, and the like. Surfactants may be ionic, usually anionic, or non-ionic. In addition, it will sometimes be desirable to pretreat the bibulous material with a fixative that can be removed by washing following application of the particles. Fixatives include polyanions, for example, polyacrylate, polycations, e.g., polybrene, water soluble organic solvents, e.g., butanol, and the like.

In the method of the invention, a first sbp member analogous to the analyte is combined in an aqueous medium with a sample suspected of containing the analyte to provide an aqueous test solution. The test solution can be preformed or it can be formed <u>in situ</u> by, for example, having the first sbp member bound to the contact portion of the bibulous material so that the test solution containing the analyte dissolves the first sbp member when the contact portion is contacted with the test solution. In addition, where the sample is an aqueous medium containing the analyte in diluted amounts, the sample can account for some or all of the aqueous medium used in forming the test solution. The primary consideration is that a test solution containing the sample and the first sbp member come in contact with the contact portion of the strip and traverse the strip through capillary action. This traversal can be upward, downward, horizontal or combinations thereof. The capillary movement along the strip causes the test solution to be carried to and through the situs. The amount of the first sbp member that becomes bound to the situs is related to the presence in the sample of the corresponding analyte in an amount at or exceeding the predetermined minimum detectible amount of that analyte. This minimum detectible amount is generally that amount above which the analyte is considered to be present. For example, for a drug one may be interested in only whether the drug is present in a certain concentration range. Although the drug might be present below that range in the sample or test solution, it would not be considered present because its concentration is not at or above the minimum detectible amount.

After the strip has been contacted with the test solution, the strip is exposed to the signal producing means. Depending on the label and the signal producing means, such exposure may be the result of irradiation, heating, or contact with chemical agents. In the latter instance the situs will be contacted with a developer solution containing the chemical agents. For this purpose the situs can be immersed in the developer solution or the contact portion of the strip can contact the developer solution which will then move to the situs by capillary action.

The signal producing system provides a detectible signal at the situs in proportion to the density of first sbp member present at the situs. The presence of the analyte in the sample will be indicated by a stronger signal at the situs that at positions on the strip adjacent to the situs.

The solvent for the test solution and/or the developer solution will normally be an aqueous medium, which may be up to about 40 weight percent of other polar solvents, particularly oxygenated solvents of from 1 to 6, more usually of from 1 to 4 carbon atoms, including alcohols, ethers and the like. Usually, the cosolvents will be present in less than about 20 weight percent. Under some circumstances depending on the nature of the sample, some or all of the aqueous medium could be provided by the sample itself.

The pH for the medium will usually be in the range of 4-11, more usually 5-10, and preferably in the range of about 6-9. The pH is chosen to maintain a significant level of binding affinity of the binding members and optimal generation of signal by the signal producing system. Various buffers may be used to achieve the desired pH and maintain the pH during the assay. Illustrative buffers include borate, phosphate, carbonate, tris, barbital and the like. The particular buffer employed is not critical, but in individual assays, one buffer may be preferred over another.

Desirably, from about 0.05 to 0.5 weight percent of a non-ionic detergent is included with the sample. Various polyoxyalkylene compounds may be employed of from about 200 to 20,000 daltons.

Moderate, and desirably substantially constant, temperatures are normally employed for carrying out the assay. The temperatures for the assay and production of a detectable signal will generally be in the range of about 4°-50°C, more usually in the range of about 10°-40°C, and frequently will be ambient temperatures, that is, about 15°-25°C.

The concentration in the aqueous test solution of analyte that may be assayed will generally vary from about $10^{-4}$ to about $10^{-15}$M, more usually from about $10^{-6}$ to $10^{-14}$M. Considerations, such as the concentration of the analyte of interest and the protocol will normally determine the concentration of the other reagents.

While the concentrations of many of the various reagents in the sample and reagent solutions will generally be determined by the concentration range of interest of the analyte, the final concentration of each of the reagents will normally be determined empirically to optimize the sensitivity of the assay over the range of interest. With certain protocols, individual reagents may be used in substantial excess without detrimentally affecting the sensitivity of the assay.

The size of the strip is dependent on several considerations. The primary consideration is to capture a sufficient amount of unbound first sbp member at the situs to give a sufficient signal so that a sensitive and accurate assay is achieved. When capillary flow is predominantly upward, the length and thickness of the strip control the amount of solution that can pass through the situs. If the transfer of a large volume of test solution is desired, the fluid capacity of the strip above the situs must be sufficient to accommodate the desired volume. If the strip is used to provide a predominantly downward flow so as to syphon the test solution, this volume requirement is not needed. Moreover, if an absorbent material is provided to contact the end of the strip not used to contact the test solution, the volume requirement is also eliminated. In general, for upward flow strips the fluid retention volume will be usually greater than 20 µL, preferably at least 50-200 µL. For downward flow strips retention volumes as low as 2-20 µL can be used but volumes of 20-200 µL are preferable.

Thickness of the strips is not critical and will normally be 0.1-2 mm, usually 0.15-1 mm, preferably 0.2-0.7 mm. Generally the minimum thickness is dictated by the strength of the material and the need to produce a readily detectible signal whereas the maximum width will be dictated by convenience of handling and cost of the reagents.

To permit conservation of reagents and provide for samples of limited size, the width of the strip will generally be relatively narrow, usually less than 20 mm, preferably less than 10 mm. Generally, the width of the strip will not be less than about 1.0 mm and will usually range from about 2 mm to 12 mm, preferably from about 4 mm to 8 mm.

The cross-sectional dimensions of the strip have been described in the preceding discussion in terms of a rectangle for the purpose of illustration and not limitation. As mentioned above, other cross sectional shapes such as circular, triagonal, oval, etc, fall equally within the scope of this invention. The dimensions thereof can be determined by those skilled in the art with reference to the disclosure herein.

The length of the strip will depend on the concentration of the analyte and practical considerations such as ease of handling and the number of situses on the strip and will be about 1 cm to 40 cm, usually about 2 cm to 25 cm, preferably about 4 to 20 cm but may be of any practical length. The structure of the strip can be varied widely and includes fine, medium fine, medium, medium coarse and coarse. In general, smaller pore size and finer material will provide slow capillary flow and efficient capture of bound conjugate on the strip. Courser more porous materials provide faster flow, but the efficiency of capture is reduced. Selection of the porosity of the material depends on the rate of binding of the components for a given assay.

The position of the situs, or situses, where a plurality of analytes are being determined, is governed by the basic principle involved in the present invention. The first sbp member will bind to the second sbp member over some length of the strip distal to the contact portion when no analyte is present. Since this distance will increase when analyte is present, it is obvious that the situs must be located an additional distance away from the contact end in order to avoid false positive assay results. If very sensitive detection is required, it may be desirable to place the situs only slightly more distant. When sensitivity is not critical may be desirable to position the situs close to the end of the strip which is opposite to the contact portion of the strip. Desirably, the situs should be at least 10 mm, preferably at least 30 mm, from the contact portion of the strip. It may be positioned any greater distance away from the end provided the test solution can pass through the situs by capillary action to capture a sufficient amount of the unbound first sbp member. In this way, the situs is "separated" from such end portion. Where several situses are used, the situses can be grouped close together or apart but must not be so close as to compromise resolution of the signal. Consequently, such situses usually should be spaced not less than 1 mm apart, preferably at least 3 mm apart.

Other reagents, which are members of the signal producing system, can vary widely in concentration depending upon the particular protocol and their role in signal production. Usually, the amount of active second sbp member on the strip will be homogeneously or uniformly bound on a portion of the strip between the contact end and the situs and is determined with reference to the predetermined minimum detectible amount of analyte. Usually, this amount will not exceed 0.5 times the maximum amount of analyte to be assayed and will not be less than about equal the minimum amount of tne first sbp member used in the assay. The minimum order concentration of first sbp member in the test solution is also determined with reference to the predetermined minimum detectible amount of analyte. This amount will usually be above the molar dissociation constant for the complex of the first and second sbp members, and the maximum amount of the first sbp member used in the assay will be less than the amount of second sbp member homogeneously bound on the strip.

In carrying out the assay, the protocol will normally involve combining in an aqueous medium the sample suspected of containing the analyte with the first sbp member to form the aqueous test solution. The sample may be derived from a wide variety of sources, such as physiologic fluids, illustrated by saliva, blood, serum, plasma, urine, ocular lens fluid, spinal fluid, etc., food products such as milk and wine, chemical processing streams, food waste water, etc.

The contact portion of the strip, usually an end portion opposite the situs, is contacted with the test solution, usually by immersion of the contact portion into the test solution. However, contact of the piece of bibulous material with the test solution can be carried out by other techniques such as by spotting the test solution on the piece of bibulous material. This technique has particular application to pieces of bibulous material that are circular, oval, sheet-like, etc. Wetting of the strip by capillary action usually is allowed to continue at least until the situs is wet. Commonly, at least half the length of the strip is wet with the test solution. When downward syphoning flow is used, or when horizontal or upward flow is assisted by an absorbent device in liquid contact with the strip, the entire strip can be wet and excess test solution can be allowed to flow through the strip.

For the most part, relatively short times are involved for the test solution to traverse the strip. Usually, the traverse of the test solution over the strip will take at least 30 sec and not more than 1 hour, more usually from about 1 min to 30 min. When an enzyme is used in the signal producing means, the development of the signal will generally range from 30 sec to 30 min, more usually from about 30 sec. to 5 min.

After the liquid has traversed the strip at least to the situs, the situs is examined for the presence of a detectible signal. Where the signal is the result of a radioactive label or the like, the signal can be detected directly at the situs. Where chemical agents form part

5687H

of the signal producing means that includes the label, the contact portion of the strip can be immersed into, or otherwise contacted with, developer solution. The resulting additional capillary flow assures contact of the situs with the test solution and washing of the situs prior to signal detection. Less desirably, when the test solution contacts the situs prior to contacting the strip with a developer solution, the situs can be contacted directly with the developer solution.

When an enzyme is used as a label, the substrate will normally be in substantial excess in the developer solution, so as not to be rate limiting (greater concentration than $K_m$). The developer solution will usually be appropriately buffered for the enzyme system.

After contacting the strip with the developer solution, the strip is contacted with any remaining members of the signal producing system not present in the developer or test solutions or present on the strip. A sufficient time is allowed to elapse prior to measuring the signal to produce an amount of the signal producing compound required to define the region of the situs in which the analyte is bound. Once the detectable signal has been produced, the presence or absence of the analyte or analytes in the sample is known.

The strip can be coated with a wide variety of materials to provide for enhanced properties. Coatings may include protein coatings, polysaccharide coatings, synthetic polymers, sugars or the like, which are used particularly to enhance the stability of the materials conjugated to the strip. These compounds can also be used for improved binding of the materials, such as antibody binding or the like.

The strip, or the situs, can be activated with reactive functionalities to provide for convalent bonding of the organic materials to be conjugated to the strip

5687H

25780-FF

0 271 204

- 33 -

such as those described in U.S. Patent No. 4,168,146, the relevent disclosure of which is incorporated herein by reference.

The second sbp member and, where desired, members of the signal producing system, can be bound to the strip by adsorption, rather than covalent bonding, as long as such binding is non-diffusive. This will involve contacting the bibulous support with a solution containing the materials to be bound to the strip and allowing the strip to dry. In general, this procedure will be useful only where the bibulous support is relatively hydrophobic or has a high surface charge, and subsequent treatment with proteins, detergents, polysaccharides, or other materials capable of blocking non-specific binding sites may be required.

In a preferred embodiment of this invention a sample suspected of containing one or more of a plurality of analytes such as drugs can be screened for the presence of one or more of such analytes. In this situation the test solution is formed by mixing together in an appropriate liquid medium the sample and a plurality of first sbp members, e.g., conjugates each comprising one of the analytes and a label. If it is only desired to know if any one of the drugs is present such as in a screening assay, the bibulous strip contains a situs identical to that described above for a single drug to which is bound a binding agent capable of specifically binding each of the first sbp members. If it is necessary to know which drugs are present, the strip contains a separate, preferably non-contiguous, situs for each drug. To each situs is bound a binding agent such as a second sbp member capable of specifically binding to a different first sbp member. In either case it is necessary to have non-diffusively and preferably homogeneously bound on the strip between the situs and

5687H

25780-FF

the contact portion a plurality of second sbp members each respectively capable of binding one of the above first sbp members.

In one embodiment of the present invention there are two analytes that are monovalent drugs. The sample suspected of containing the drugs is mixed with conjugates of an enzyme with one of each of the drugs to form the aqueous test solution. The bibulous strip has homogeneously bound to a lower segment antibodies for each of the drugs. An upper segment of the strip is provided in contact with the lower segment. It has no antibody over most of the segment but has two thin bands, each produced by binding one of the antibodies to a separate situs. The situses are at the end opposite the contact portion of the strip. As a consequence, the drugs and conjugates are captured prior to the test solution reaching a situs when the contact portion is contacted with the test solution. The amount of homogeneously bound antibodies for the drugs are selected to bind all of each conjugate. When the sample and the conjugate are mixed together to form the test solution and a drug is present in the sample, a competition occurs for antibody on the strip as the solution traverses the strip. The more drug in the sample, the less conjugate becomes bound by antibody for the drug. Unbound conjugate moves along and binds to the bibulous strip until it reaches the upper segment. It then moves along the segment until it reaches the situs corresponding to that drug where it becomes bound due to binding with the antibody at the situs. If no drug is present in the sample, then all of each of the conjugates will be bound by antibodies for the drugs and captured prior to reaching a situs. If both drugs are present each of the conjugates will become bound to its corresponding situs. In subsequent development of the test strip, the presence

5687H

of a given drug in the sample will be indicated by production of a signal at its corresponding situs. The test solution can traverse all or part of the strip by capillary action. If the test solution is allowed to traverse the strip through the situs, the strip can subsequently be immersed in the developer solution.

In a variant of the above-described embodiment, the volume of the test solution may be sufficient to permit it to traverse only a portion of the strip such that the fluid capacity at the dry portion of the strip is at least as great as the fluid capacity of the portion from the contact portion through the situs. The contact portion of the strip is next contacted with the developer solution. The developer solution moves along the strip through the situs by capillarity. In doing so, the developer solution causes the remainder of the test solution to move through the small situs. If one or more of the analytes is present in the test solution, a signal is generated.

In another embodiment of the present invention a sample suspected of containing two drugs, e.g. morphine and tetrahydrocannabinol, is analyzed. The sample suspected of containing the drugs is mixed with two conjugates to form a test solution. Each conjugate comprises one of the drugs bound to an enzyme, e.g., horseradish peroxidase, to each of which is also bound a different member of a pair of low molecular weight molecules, e.g., fluorescein and biotin. A bibulous strip has homogeneously bound to a lower segment antibodies for each of the drugs. An upper segment of the strip is provided in liquid receiving relationship with the lower segment. The portion of the strip between the upper and lower segments is free of specific binding members. The upper segment is comprised of two bands or situses adjacent to, and preferably separated from, one another. Each band has a binding agent for one of the low molecular weight components of the conjugates, e.g., antibodies for fluorescein or avidin. The end of the strip opposite the situses is contacted with the test solution. As a consequence, the drugs and conjugates are captured prior to the test solution reaching a situs. The amount of homogeneously bound antibodies are selected to bind all of each conjugate. When the sample and the conjugates are mixed together to form the test solution and one or more of the drugs is present in the sample, a competition occurs for the antibody on the strip as the solution traverses the strip. The more of one of the drugs in the sample, the less of one of the corresponding conjugates becomes bound by antibody for the drug. Unbound conjugate moves along and binds to the bibulous strip until it reaches the upper segment. It then moves along the segment until it reaches the situs corresponding to that drug where it becomes bound due to binding with the binding agent at the situs. If no drug is present in the sample, then all of each of the conjugates will be bound by antibodies for the drugs and captured prior to reaching a situs. If both drugs are present each of the conjugates will become bound to its corresponding situs. In subsequent development of the test strip, the presence of a given drug in the sample will be indicated.

As a matter of convenience, the present device can be provided in a kit in packaged combination with predetermined amounts of reagents for use in assaying for an analyte or a plurality of analytes. Where an enzyme is used as the label, the reagents will include enzyme labeled analyte and the developer solution can contain substrate for the enzyme or precursors therefor including any additional substrates, enzymes and cofactors and any reaction partner of the enzymic product required to

5687H                                                        25780-FF

contained three horizontal lines of anti-benzoylecgonine, anti-methadone and anti-morphine coated polyacrylamide beads respectively (prepared in Example 2). The bead lines were 0.5 cm apart. The lowest line was 0.5 cm above the top of the lower strip. Both paper strips were coated with protein (ovalbumin) and detergent to reduce nonspecific binding and improve wicking quality.

### Example 4
### Assay for Benzoylecgonine, Methodone, and Morphine

1. The enzyme reagent was a mixture consisting of horseradish peroxidase (HRP)-benzoylecgonine (1.27 µg/mL), HRP-methadone (0.50 µg/mL) and HRP-morphine (0.39 µg/mL) in the following buffer:

100mM phosphate pH 7.0
200mM NaCl
2 mg/mL BgG (bovine gamma globulin)
0.05% QS-15 (from Sigma Chemical Co., St. Louis, Mo - trade mark)
100 µg/mL glucose oxidase

2. The enzyme developer consisted of:
11 mM phosphate pH 6.5
2 mg/mL polyvinyl alcohol 20/30
400 µg/mL 4-chloro-1-naphthol
50 mM βD-(+)-glucose

3. Assay Protocol
The assay was performed at ambient temperature; 80µL of enzyme reagent was dispensed into a 13x100 mM test tube and 20 µL of buffer (same as buffer in section 1 above) sample was added to form a test solution. The composite antibody bead test strip from Example 3 was placed in the enzyme reagent test

5687H

tube. All the test solution was allowed to wick up the composite strip (3-4 minutes). The strip was transferred to a second test tube containing 3.0 mL of enzyme developer. This volume was sufficient to completely immerse only the bottom portion of the composite strip.

4. Results

In a positive sample the entire bottom portion of the strip turned purple and one or more of the bead lines also developed color within 3-4 minutes allowing for specific drug identification. For example, in a sample containing cocaine the anti-cocaine bead line developed color, but the anti-methadone and anti-morphine bead lines remained colorless. In a sample containing both cocaine and methadone, both the anti-cocaine and anti-methadone bead lines developed color, but the anti-morphine bead line remained colorless. In a sample with none of the three analytes all the bead lines remained colorless and only the very lowest portion of the bottom part of the strip was colored.

The above results indicate that the presence of, and identity of, one or more of a plurality of analytes can be determined utilizing the method and device of the present invention.

Example 5
Assay for Morphine and Phenobarbital
1. Coated Paper:
a) A roll of Whatman 31 ET chromatography paper was activated with carbonyldiimidazole (CDI). CDI activated paper was dipped into a tray containing 50-200 ml of 0.1- 2.0 mg/ml of antibodies (1.6 mg/ml of antibody for morphine and 0.4 mg/ml of antibody for phenobarbital), 0.1 mg/ml of glucose oxidase amine (GO-NH$_2$), and the total protein was bulked to 2.1 mg/ml using non-immuno sheep immunoglobulin G (IgG), in 0.1 M NaPO$_4$, 0.2 M NaCl, pH 7.0 phosphate buffer solution (PBS). The paper was removed from the solution and incubated for 1-3 hours. Unreacted CDI sites were blocked by incubating the paper in a 0.3M ethanolamine solution for 16 hours. The paper was washed with deionized water. The paper was dipped into 200-400 ml of preservative solution (0.6% polyvinyl alcohol 30/20 (PVA) and was dried in air dryer at 65° for 7-15 minutes.

b)    In a manner similar to that described above, avidin coated paper and paper coated with antibody for theophylline were prepared.

c)    A sheet of S&S GB002 paper was dipped into a 2.0 mg/ml aqueous bovine serum albumin (BSA)/PBS solution and was then blotted and dried at 65°C for 7 minutes.

2.    Peroxidase Conjugation:

Horseradish peroxidase (275 mg, HRP) was dissolved in 5 ml 10 mM NaOAc pH 4.5. The mixture was dialysed overnight. A volume containing 180 mg HRP (~4.5ml) was diluted to 9 ml with 10 mM NaOAc in a 20 ml stirring flask and cooled to 0-4°C. While stirring the HRP, 1.8 ml 0.1 M NaIO$_4$ in distilled H$_2$O was added dropwise. After the addition was complete, stirring was stopped and the reaction was allowed to proceed 30 minutes at 0°C. 1.2 ml of 1 M glycerol in H$_2$O was added to the stirring solution. Stirring was stopped and the reaction was allowed to proceed for 30 minutes at 0°C. The reaction mixture was dialyzed at 4°C overnight. The flask was placed in a dessicator under vaccuum in the presence of P$_2$O$_5$ until HRP-amine was characterized.

Into a 5ml stirring flask were placed 300 mg 3-(3'-carboxypropyl)-1-methylxanthine, 150 mg N-hydroxysuccinimide (NHS), and 247.5 mg EDCI. 3.6ml 1 M

5687H

oxybis (ethylamine) dihydrochloride was placed in 0.5 M $Na_2CO_3$ in a 20ml stirring flask and cooled to 0°. To the stirring mixture was added the oxidized HRP over about 10 minutes. The reaction was allowed to proceed 3 hours at 0-5°C without stirring. Then, 1.75 ml 1 M $NaBH_4$ in $dH_2O$ was added to the stirring solution and the reaction was allowed to proceed for 2 hours at 0-5°C. The HRP-amine reaction mixture was dialyzed. In a similar manner, the NHS esters of phenobarbital, morphine, and biotin were formed.

To the HRP-amine from above (5mg/ml) was added morphine NHS ester at 0°C followed by the NHS ester of biotin. The conjugate was dialysed and either stored at -20°C or sterile filtered into autoclaved vials at 4°C.

Conjugates of HRP with morphine and biotin, and of HRP with phenobarbital and theophylline were prepared in the above manner.

3.  Enzyme Reagent:

Buffer:  100mM $NaH_2PO_4$, pH 7.0, 200 mM NaCl

Additives:

| | |
|---|---|
| Bovine gamma globulin (BGG) | 2.0 mg/ml |
| Iodoacetamine | 0.2 mg/ml |
| Benzamidine HCl | 0.8 mg/ml |
| ANS (8-anilino-1-naphthalene sulfonic acid) | 0.2 mg/ml |
| Gentamycin Sulfate | 0.05 mg/ml |
| Sodium Benzolate | 1.0 mg/ml |
| Triton QS-15     (trade mark) | 0.5 mg/ml |

Enzyme Conjugates:

| | |
|---|---|
| Phenobarbital-HRP-Theophylline conjugate | 0.4 ug/ml |
| Morphine-HRP-Biotin conjugate | 0.4 ug/ml |

5687H

25780-FF

4.   Developer:

Buffer: 10 mM $NaH_2PO_4$ pH 6.5
Additives: 2% polyvinyl alcohol (20/30)
Substrates:   400 ug/ml   4 Cl-1-Naphthol
                    50-100 mM glucose

5.   Strip:

The antibody coated paper from 1.a) above was laminated onto 3M double side adhesive (#491) and was cut into 30x150 mm strips.  This paper was then placed horizontally onto a 10 ml thick polystyrene plastic backing (90x150 mm) at the bottom portion.  A 5x150 mm strip of the paper of 1.c) above laminated onto 3M adhesive was placed immediately above the strip of 1.a) paper.  Immdediately above the 1.c) paper was placed a strip (5x150 mm) of theophylline coated paper of 1.b) laminated to 3M adhesive followed next by a 5x150 mm strip of the laminated paper of 1.c).  A 5x150 mm strip of avidin-coated paper of 1.b) laminated to the 3M adhesive was placed next on the polystyrene backing above the paper of 1.c).  Finally, a 45x150 mm strip of laminated S&S GB004 paper was placed at the top of the backing above the avidin-coated paper.  The polystyrene sheet with the attached strips was then cut vertically into strips (5x90 mm), which were employed in the assay. The so formed strips had segments, each corresponding to a respective paper prepared in section 1 above.

6.   Protocol:

The strip was put into a 13X100mm test tube containing 0.5 ml of enzyme reagent spiked with either 6μL of PBS or 80 μg/ml phenobarbital or 83 μg/ml of morphine/ethanol solution, to form the test solution. The test solution was allowed to wick along the strip for 5-6 minutes.  The strip was placed into a test tube containing 2 ml of developer which was allowed to wick for 7-10 minutes. The strip was removed from the developer solution and the strip including the situses were examined for color development.

7. Results:

For a negative sample, color developed only on the bottom portion of the strip. For a positive morphine sample, color developed on both the bottom part of the strip and the situs that contained avidin which captured the morphine-HRP-biotin conjugate. For a positive phenobarbital sample color developed on both the bottom part of the strip and the situs that contained the theophylline antibody which captured the phenobarbital-HRP-theophylline congugate. For a positive morphine and phenobarbital sample, color developed on the bottom part of the strip and at both situses.

The above results indicate that the presence of, and identity of, one or more of a plurality of analytes can be determined utilizing the method and device of the present invention.

The invention has been described in detail with particular reference to the above embodiments. It will be understood, however, that variations and modifications can be effected within the spirit and scope of the invention.

5687H                                                25780-FF

CLAIMS

1.     A method for determining the presence of an analyte in a sample suspected of containing said analyte, which analyte is a member of a specific binding pair ("sbp member") consisting of ligand and its complementary receptor, which method comprises -

(a)     contacting, with a test solution containing said sample and a first sbp member analogous to said analyte, a contact portion of a piece of bibulous material capable of being traversed in at least one direction by said test solution by capillary migration, said bibulous material containing a second sbp member capable of binding said analyte and said first sbp member non-diffusively bound thereto at least at a portion thereof between said contact portion and a small situs on said bibulous material separated from said contact portion, the surface area of said situs being substantially less than that of said piece of bibulous material, said situs being capable of binding said first sbp member,

(b)     allowing at least a portion of the test solution to traverse said piece of bibulous material by means of capillary migration and thereby contact said situs,

(c)     detecting said first sbp member at said situs.


2.     The method of Claim 1 wherein said first sbp member is a conjugate of said analyte, a label, and a third sbp member and receptor for said third sbp member is non-diffusively bound to said situs.


3.     The method of Claim 1 or 2 wherein a high density of said second sbp member is provided at said situs compared to portions of said strip adjacent to said situs.

4.    The method of any one of the preceding Claims wherein said second sbp member at said situs is bound to particles.

5.    The method of any one of the preceding Claims wherein said first sbp member is conjugated to a label.

6.    The method of Claim 5 wherein said label is an enzyme.

7.    The method of any one of the preceding Claims wherein the analyte is a drug, a drug analog is conjugated to a label, and said second sbp member is antibody to said drug.

8.    The method of any one of the preceding Claims wherein the small situs is a band transverse to the direction of traversal of said test solution along said piece.

9.    The method of any one of the preceding Claims wherein the signal produced at the small situs has a sharp-edged distinctive pattern that provides a sharp contrast to signal produced at adjacent sites on said piece of bibulous material when analyte is present in said test solution.

10.    The method of Claim 6 wherein a second enzyme is bound uniformly to said piece at least over an area including said situs and the portions of the piece adjacent to said situs, the enzymes being related in that the product of one enzyme is the substrate for the other.

5687H

11.   The method of any one of the preceding Claims for identifying which of a plurality of analytes are present in said test solution, wherein a plurality of second sbp members are each non-diffusively bound to said strip and each of said second sbp members is respectively capable of specifically binding a corresponding first sbp member and wherein a plurality of situses are employed, each respectively capable of binding a first sbp member.

12.   The method of any one of the preceding Claims wherein said test solution is allowed to traverse said piece through said situs in step (b).

13.   The method of any one of the preceding Claims wherein a high density of a binding agent capable of binding said first sbp member is provided at said situs compared to portions of said piece adjacent to said situs.

14.   The method of any one of the preceding Claims wherein said first sbp member is detected at said situs by directly detecting a detectible signal at said situs.

15.   A method according to Claim 1 for determining the presence of an analyte in a sample suspected of containing said analyte, which comprises -
     (a)   contacting, with a test solution containing said sample and an analyte analog conjugated to an enzyme, the end portion of a strip of bibulous material capable of being traversed by said test solution by capillary migration, said strip containing antibody for said analyte non--diffusively bound thereto at least at a portion thereof between a small situs on said strip separated from said end portion and said end portion, the surface area of said situs being substantially less than that of said strip, said situs having non-diffusively bound thereto a binding agent for said conjugated analyte analog at a density higher than the density of said antibody bound to said strip adjacent to said situs,

(b)     allowing at least a portion of the test solution to traverse said strip by capillary migration,

(c)     contacting said strip with a developer solution containing members of a signal producing system other than said enzyme to provide contact of said developer solution with said situs said signal producing system being capable of producing a detectible signal at said situs in relation to the amount of analyte in the test solution, and

(d)     comparing said detectible signal at said situs with the signal detectible at a portion of the strip adjacent to said situs.

16.     The method of Claim 15 for determining a plurality of analytes in said sample wherein a specific antibody to each analyte is non-diffusively bound to said strip and wherein a plurality of situses are employed, each respectively having a receptor specific for conjugated analyte analog non-diffusively bound thereto.

17.     The method of Claim 15 or 16 wherein said binding agent is an antibody for an analyte.

18.     The method of Claim 15, 16 or 17 wherein said small situs is a band traverse to the direction of traversal of said sample along said strip.

19.     The method of any one of Claims 15 to 18 wherein the signal produced at the small situs has a sharp-edged distinctive pattern that provides a sharp contrast to signal produced at adjacent sites on said strip when analyte is present in said test solution.

20. A device for determining the presence of an analyte in a test solution comprising a sample suspected of containing said analyte and a first sbp member, which analyte is a member of a specific binding pair, said device comprising a bibulous strip capable of traversal by said test solution by capillary migration, said strip having a contact portion for contacting said test solution and a second sbp member complementary to said analyte non-diffusively bound to said strip at least at a portion thereof between said contact portion and a small situs on said strip separated from said contact portion, the surface area of said situs being substantially less than that of said strip, said situs having non-diffusively bound thereto a binding agent capable of binding said first sbp member.

21. A device according to Claim 20 for determining the presence of an analyte in a test solution comprising a sample suspected of containing said analyte and an analyte analog, said device comprising a bibulous strip capable of traversal by said test solution by capillary migration, said strip having a contact portion for contacting said test solution and an antibody for said analyte non-diffusively bound to said strip at least at a portion thereof between said contact portion and a small situs on said strip separated from said contact portion, the surface area of said situs being substantially less than that of said strip, said situs having a non-diffusively bound thereto a binding agent capable of binding said analyte analog.

22. The device of Claim 21 for determining the presence of a plurality of analytes in said test solution wherein each of a set of antibodies is respectively

5687H                                                      25780-FF

non-diffusively bound to said strip and to each of different small situses on said strip.

23. A kit for use in determining the presence of an analyte in a test solution, said kit comprising in a packaged combination--

(a) the device of Claim 20

(b) said first sbp member as part of a signal producing system,

(c) other members of signal producing system for producing a detectible signal in the presence of said analyte as required, and

(d) ancillary materials as required.

24. A method according to Claim 1 for determining the presence of one or more of a plurality of analytes in a sample suspected of containing said analytes, each analyte being a member of a specific binding pair ("sbp member") consisting of ligand and its complementary receptor, which method comprises -

(a) contacting, with a test solution containing said sample and a plurality of first sbp members each respectively analogous to one of said analytes, a contact portion of a piece of bibulous material capable of being traversed in at least one direction by said test solution by capillary migration, said bibulous material containing a plurality of second sbp members each respectively capable of binding one of said analytes and said first sbp members non-diffusively bound thereto at a portion thereof at least between said contact portion and one or more of a plurality of small situses on said bibulous material separated from said contact portion, the surface area of each situs being substantially less than that of said piece of bibulous

5687H

25780-FF

material, each situs being capable of binding at least one of said first sbp members.

(b) allowing at least a portion of the test solution to traverse said piece of bibulous material by means of capillary migration and thereby contact said situs,

(c) detecting one or more of said first sbp members at one or more of said situses.

25. The method of Claim 24 wherein said first sbp member is a conjugate comprising one of said analytes, an enzyme, and a third sbp member and said binding agent is a receptor for said third sbp member.

26. The method of Claim 24 wherein each of said first sbp members is conjugated to a label, the label is an enzyme and a second enzyme is bound uniformly to said piece at least over an area including said situses and the portions of the piece of bibulous material adjacent to said situses, the enzymes being related in that the product of one enzyme is the substrate for the other.

27. A device according to Claim 20 for determining the presence of one or more of a plurality of analytes in a test solution, each analyte being a member of a specific binding pair, said device comprising a piece of bibulous material capable of traversal in at least one direction by said test solution by capillary migration, said bibulous material having a contact portion for contacting said test solution and one or more of a plurality of second sbp members each complementary to one of said analytes non-diffusively bound to said bibulous material at least between said contact portion and one or more small situses on said bibulous material separated from said contact portion, the surface area of each situs being substantially less than that of said bibulous material, each situs having non-diffusively bound thereto at least one binding agent capable of binding said first sbp member.

28. The device of Claim 27 wherein said piece of bibulous material is constructed in segments.

29. A kit according to Claim 23 for use in determining the presence of an analyte in a test solution, said kit comprising in a packaged combination--
    (a) the device of Claim 27
    (b) said first sbp member as part of a signal producing system,
    (c) other members of signal producing system for producing a detectible signal in the presence of said analyte as required, and
    (d) ancillary materials as required.

30. A method according to Claim 1 for determining the presence of one or more of a plurality of analytes, each analyte being a member of a specific binding pair (sbp) consisting of ligand and its complementary receptor, in a sample suspected of containing one or more of said analytes, which comprises -
    (a) contacting, with a test solution containing said sample and one or more of a plurality of conjugates each comprising one of said analytes, a label, and an sbp member, a contact portion of a piece comprised of bibulous material capable of being traversed in at least one direction by said test solution by capillary migration, said bibulous material containing non-diffusively bound to a portion thereof at least between said contact portion and one or more situses separate from said contact portion a plurality of first receptors each respectively capable of binding to one of said analytes, said bibulous material further containing non-diffusively bound thereto at one or more situses one or more of a plurality of second receptors each respectively capable of binding to a corresponding sbp member of said conjugate, the surface area of said situs being less than that of said bibulous material, said situs being separated from said portion of said bibulous material containing one or more first receptors,

(b) allowing at least a portion of said test solution to traverse said bibulous material by capillary migration and thereby contact one or more situses, and

(c) examining said situses for the presence of one or more of said conjugates.

31. The method of Claim 30 wherein said analytes are morphine and tetrahydrocannabinol, said conjugates are morphine-horseradish peroxdase-fluorescein and tetrahydrocannabinol-horseradish peroxidase-biotin, said first receptors are antibodies for morphine and antibodies for tetrahydrocannabinol, and said second receptors are antibodies for fluorescein and avidin.

32. A device according to Claim 20 for determining the presence of one or more of a plurality of analytes, each analyte being a member of a specific binding pair (sbp) consisting of ligand and complementary receptor, in a test solution comprised of (i) one or more conjugates comprising one of said analytes, a label, and an sbp member and (ii) a sample suspected of containing the analyte, said device comprising -

a piece comprised of bibulous material capable of traversal in at least one direction by said test solution by capillary migration, said bibulous material having a contact portion for contacting said test solution and one or more of a plurality of first receptors each respectively capable of binding to one of said analytes non-diffusively bound to said bibulous material at a portion lying at least between said contact portion and one or more situses, the surface area of each situs being less than that of said bibulous material, each situs having non-diffusively bound thereto one or more of a plurality of second receptors each respectively capable of binding an sbp member, said situs being separated from said portion of said strip containing said first receptor.

33. A kit according to Claim 23 for use in determining the presence of one or more of a plurality of analytes in a test solution, comprising in a packaged combination -

(a) one or more of a plurality of conjugates each comprising one of said analytes, a label, and a member of a specific binding pair, and

(b) the device of Claim 32.

35. A device for determining the presence of an analyte in a test solution, said device comprising a piece of bibulous material containing a segment to which a member of a specific binding pair (sbp) complementary to said analyte is homogeneously and non-diffusively bound, characterised by having a situs on said bibulous material, said situs having non-diffusively bound thereto a binding agent capable of binding an analog of said analyte.

36. A kit for use in determining the presence of one or more of a plurality of analytes in a test solution, comprising in a packaged combination -

(a) one or more of a plurality of conjugates each comprising one of said analytes, a label, and a member of a specific binding pair, and
(b)    the device of Claim 35.

5687H

25780-FF